# EUROPÄISCHE PATENTSCHRIFT

(11) **EP 1 842 487 B1**
(45) Veröffentlichungstag und Bekanntmachung des Hinweises auf die Patenterteilung: **19.08.2009**
(21) Anmeldenummer: 06007103.2
(22) Anmeldetag: 04.04.2006
(51) Int. Cl.: A61B 6/02

(54) **Stereoskopische Röntgenverfolgung bewegter Objekte im Rahmen der Radiotherapie und Radiochirurgie**
Stereoscopic X-ray imaging of moving objects for radiotherapy and radiosurgery
L'imagerie par rayons X stéréoscopique des objects mobiles pour la radiothérapie et la radiochirurgie

(43) Veröffentlichungstag der Anmeldung: 10.10.2007
(73) Patentinhaber: BrainLAB AG, 85622 Feldkirchen (DE)
(72) Erfinder: Erbel, Stephan, 81677 München (DE)
(74) Vertreter: Rögner, Jürgen

(56) Entgegenhaltungen:
- EP-A- 1 479 411
- US-A- 5 207 223
- US-A1- 2002 115 923
- US-A1- 2002 122 530
- US-A1- 2003 048 868
- US-A1- 2004 037 390
- US-A1- 2004 042 583

## Beschreibung

Die Erfindung betrifft die nicht diagnostische, stereoskopische Röntgenverfolgung bewegter Objekte im Rahmen der Radiotherapie und Radiochirurgie. Speziell betrifft sie eine Röntgenverfolgung, bei der mit zwei Röntgenröhren wiederholt Röntgenbilder eines Objekts entlang zweier verschiedener, sich unter einem bekannten Winkel schneidender Sichtlinien durch das Zielgebiet eines Bestrahlungsgerätes aufgenommen werden.

Eine Echtzeit-Röntgenverfolgung bewegter Ziele, beispielsweise implantierter Tracking-Marker, ist im Rahmen der Radiochirurgie bzw. Strahlentherapie grundsätzlich bekannt. So beschreibt beispielsweise die US 5,207,223 ein Verfahren und ein Gerät zum selektiven Bestrahlen eines Ziels innerhalb eines Patienten, wobei wiederholt mit zwei in einem Winkel zueinander angeordneten Röntgen-Bildaufnahmesystemen Bilder des Zielgebiets und eines in dessen Nähe implantierten Markers gemacht werden, um in Echtzeit feststellen zu können, wo sich der Marker und damit das Zielgebiet zu jedem Zeitpunkt realiter befinden. Dies ist besonders bei Zielen oder Zielgebieten wichtig, die sich (beispielsweise mit der Atembewegung des Patienten) bewegen. Bei diesem Verfahren werden beide Röntgen-Bilderfassungseinheiten zur gleichen Zeit oder im Wesentlichen gleichzeitig betätigt, damit die beiden Sichtlinien für ein sich bewegendes Objekt sich im dreidimensionalen Raum zeitgleich schneiden und für einen bestimmten Zeitpunkt die erfasste Position des Markers bzw. des Zielgebiets bestimmen können.

Bei den hohen verwendeten Abtastraten, die notwendig sind, um die Bewegung genau zu verfolgen, wird der Patient bei solchen Systemen einer relativ hohen Strahlenbelastung ausgesetzt, wenn beide Röntgensysteme jeweils gleichzeitig aktiviert werden.

Es ist eine Aufgabe der vorliegenden Erfindung, eine stereoskopische Röntgenverfolgung für bewegte Objekte im Rahmen der Radiotherapie und Radiochirurgie zu schaffen, welche die Strahlenbelastung des Patienten reduziert.

Diese Aufgabe wird erfindungsgemäß durch ein nicht diagnostisches, stereoskopisches Röntgenverfolgungsverfahren gemäß dem Anspruch 1 sowie durch eine stereoskopische Röntgenverfolgungsvorrichtung gemäß dem Anspruch 9 gelöst. Die Unteransprüche definieren bevorzugte Ausführungsformen der Erfindung.

Die vorliegende Erfindung zeigt auf, wie ein bewegtes Objekt in einem Patienten verfolgt werden kann, also beispielsweise ein Objekt, das seine Lage mit der Atembewegung eines Patienten verändert. Wenn vor der Therapie mit bildgebenden Verfahren (CT, MR, etc.) dreidimensionale Bildaufnahmen des Patienten im Gebiet um das Behandlungsziel erstellt werden, auf denen auch das Objekt zu sehen ist, können diese bekannten Patientenstrukturen durch die erfindungsgemäße Echtzeit-Verfolgung des Objektes bzw. eines Markers oder einer Landmarke immer an ihrem gerade aktuellen Ort im dreidimensionalen Raum bestimmt werden. Es ist deshalb möglich, die Bestrahlung an die Bewegung des Objektes anzupassen (Bewegung des Patienten, Nachführen der Strahlvorrichtung, Gating = An- und Abschalten des Strahls zu geeigneten Zeitpunkten) und somit gesundes Gewebe zu schonen und krankes Gewebe gezielt zu bestrahlen.

Zu diesem Zweck beschreibt die vorliegende Erfindung ein nicht diagnostisches, stereoskopisches Röntgenverfolgungsverfahren zur Verfolgung bewegter Objekte im Rahmen der Radiotherapie und Radiochirurgie, bei dem mit zwei Röntgenröhren wiederholt Röntgenbilder eines Objekts entlang zweier verschiedener, sich unter einem bekannten Winkel schneidender Sichtlinien durch das Zielgebiet eines Bestrahlungsgerätes aufgenommen werden, dadurch gekennzeichnet, dass
a) die Ansteuerung der Röntgenröhren so erfolgt, dass abwechselnd mit einer der beiden Röntgenröhren jeweils ein Bild erzeugt wird; und computerunterstützt
b) eine Fläche bestimmt wird, die aufgespannt wird durch
   - die Sichtlinie von einer ersten Röntgenröhre zum Objekt in einem zu einem früheren Zeitpunkt aufgenommenen Bild und
   - die Sichtlinie von der ersten Röntgenröhre zu dem Objekt in einem zu einem späteren Zeitpunkt aufgenommenen Bild;
c) ein Schnittpunkt bestimmt wird aus
   - einer Sichtlinie von der zweiten Röntgenröhre zu dem Objekt in einem Bild, das zu einem Zeitpunkt aufgenommen wird, der zwischen dem früheren und dem späteren Zeitpunkt liegt, und
   - der aufgespannten Fläche;
d) eine weitere Fläche bestimmt wird, wobei der spätere Zeitpunkt zu einem neuen früheren Zeitpunkt wird, und wobei die weitere Fläche aufgespannt wird durch
   - die Sichtlinie von der ersten Röntgenröhre zum Objekt in einem zu dem neuen früheren Zeitpunkt aufgenommenen Bild und
   - die Sichtlinie von der ersten Röntgenröhre zu dem Objekt in einem zu einem neuen späteren Zeitpunkt aufgenommenen Bild;
e) ein weiterer Schnittpunkt bestimmt wird aus
   - einer Sichtlinie von der zweiten Röntgenröhre zu dem Objekt in einem Bild, das zu einem Zeitpunkt aufgenommen wird, der zwischen dem neuen früheren und dem neuen späteren Zeitpunkt liegt, und
   - der weiteren aufgespannten Fläche
f) die räumliche Verbindungsgerade R12, welche die Schnittpunkte verbindet, berechnet wird;
g) die Minimaltransversale zwischen der Verbindungsgeraden R12 und der Sichtlinie des Objektes zum neuen späteren Zeitpunkt errechnet wird; und
h) aus dem Schnittpunkt der Minimaltransversalen und der Sichtlinie zum neuen späteren Zeitpunkt die dreidimensionale Position des verfolgten Objektes approximiert wird.

Einfacher und allgemeiner ausgedrückt werden bei dem erfindungsgemäßen Verfahren mit zwei Röntgenröhren abwechselnd und wiederholt Röntgenbilder eines Objekts entlang zweier verschiedener Sichtlinien aufgenommen, und durch Flächen- und Schnittpunktsbestimmungen wird eine extrapolierte Objektbahn ermittelt. Die Minimaltraverse von der Objektbahn auf eine aktuelle Sichtlinie wird ermittelt und an ihrem Auftreffpunkt auf die Sichtlinie wird die dreidimensionale Position des verfolgten Objektes approximiert.

Dieses Verfahren macht es also möglich, die Röntgenbilder in einem zeitlichen Abstand zu erfassen und mit Hilfe einer Approximation trotzdem sehr genau in Echtzeit die Lage des Objekts zu bestimmen, und zwar obwohl das Objekt sich während der Zeit zwischen zwei Röntgenvorgängen bewegen wird. Weil die Röntgenaufnahmen mit den beiden im System verwendeten Röntgenröhren abwechselnd erstellt werden, wird bei jedem Röntgen-"Schuss" nur eine Röhre aktiviert und die Strahlungsbelastung des Patienten kann um bis zu 50 % verringert werden, ohne die zeitliche Auflösung der Verfolgung, insbesondere entlang der Hauptbewegungsachse des Objektes, zu vermindern. Mit anderen Worten ersetzt die vorliegende Erfindung einen Hauptteil der zur Verfolgung notwendigen Röntgenaufnahmen durch eine geeignete und intelligente Verwendung von Bild- bzw. Lagedaten, die für das Objekt und die Röntgenaufnahmen (wegen im Raum bekanntet Sichtlinien) ohnehin vorhanden sind. Eine solche intelligente Datenverwertung bringt aber noch weitere Vorteile mit sich, wie sie im Folgenden erörtert werden.

Die Streustrahlung entlang einer der Bildaufnahmeachsen eines der Röntgensysteme beeinträchtigt die Bilderstellung des anderen Röntgensystems nicht, weil die beiden Systeme nicht zeitgleich aktiviert werden. Dies gilt insbesondere dann, wenn die Auslesezeiten an den jeweiligen Detektoren ebenfalls zeitversetzt sind. Der maximale Energieverbrauch des Gesamtsystems wird verringert, weil es nicht länger notwendig ist, beide Röntgenröhren gleichzeitig zu aktivieren. Die Gesamt-Nennleistung des Systems wird entsprechend reduziert. Die Erwärmung der Röntgenröhren, die für das Echtzeit-Tracking über längere Zeiträume eine funktionelle Einschränkung darstellt, wird verringert, wenn jede Röhre im Verhältnis zu herkömmlichen Systemen nur halb so oft betätigt wird. Falls notwendig, kann die zeitliche Auflösung des Verfolgungssystems bzw. Trackingsystems verdoppelt werden, und zwar indem jedes Röhren/Detektor-Paar mit seiner maximal möglichen Frequenz betätigt wird.

Speziell der erstgenannte Vorteil, nämlich die Dosisreduktion für den Patienten, ist von hoher Wichtigkeit für Radiotherapie- bzw. Radiochirurgieanwendungen, bei denen die Verfolgung über lange Zeitabschnitte durchgeführt werden muss. Über die Zeit einer vollständigen Behandlung erreicht nämlich die Hautdosis, die durch die Tracking-Röntgenbestrahlung appliziert wird, oftmals kritische Niveaus.

Gemäß einer Ausführungsform der vorliegenden Erfindung wird der zeitliche Abstand zwischen dem Erzeugen eines Bildes mit der ersten und zweiten Röntgenröhre so eingestellt, dass in einem regelmäßigen Zeitabstand jeweils abwechselnd ein Bild mit der ersten und zweiten Röhre erzeugt wird. Das verfolgte Objekt kann ein Markierungsimplantat sein, insbesondere ein Implantat, das sich korreliert zur Atembewegung bewegt. Es kann aber auch eine röntgenabbildbare Körperstruktur oder Körperlandmarke sein, insbesondere eine Struktur oder Landmarke, die sich korreliert zur Atembewegung bewegt. Aus der Bewegung des Implantats bzw. der Struktur/Landmarke kann dann auf die Bewegung der umliegenden Körperstrukturen geschlossen werden, die als dreidimensionaler Datensatz (CT, MR etc.) in einem dem erfindungsgemäßen System angeschlossenen Navigationssystem hinterlegt sind, so dass auch der Echtzeit-Ort des tatsächlichen Bestrahlungsziels immer bekannt ist.

Die erfindungsgemäße Objektverfolgung arbeitet sehr genau, wenn die Minimaltransversale, also das kürzeste Lot von der Verbindungsgeraden auf die Sichtlinie, keine zu große Länge aufweist. Deshalb wird bei einer bevorzugten Ausführungsvariante die Länge der Minimaltransversalen mit einem vorgegebenen Schwellwert verglichen, wobei beim Überschreiten des Schwellwertes ein Zustand der Ungenauigkeit angenommen wird. Nunmehr kann einerseits eine Verfahrensanpassung vorgenommen werden, insbesondere eine Erhöhung der Abtastrate, die durch eine Verkleinerung der Zeitabstände zwischen den Bildern realisiert wird. Andererseits besteht auch die Möglichkeit, eine Fehlermeldung auszugeben, die den Verwender auf einen solchen Ungenauigkeitszustand hinweist.

Die Erfindung betrifft auch ein Programm, das, wenn es auf einem Computer läuft oder in einem Computer geladen ist, den Computer veranlasst, ein Verfahren durchzuführen, wie es hierin in verschiedenen Ausführungsformen beschrieben wird. Ferner betrifft sie ein Computerprogramm-Speichermedium, das ein solches Programm aufweist.

Gemäß einem anderen Aspekt der vorliegenden Erfindung bezieht sich diese auf eine stereoskopische Röntgenverfolgungseinrichtung zur Verfolgung bewegter Objekte im Rahmen der Radiotherapie und Radiochirurgie, mit zwei Röntgenröhren (1, 2), die wiederholt Röntgenbilder eines Objekts (O) entlang zweier verschiedener, sich unter einem bekannten Winkel schneidender Sichtlinien durch das Zielgebiet eines Bestrahlungsgerätes aufnehmen, gekennzeichnet durch eine Steuerungseinrichtung, welche die Röntgenröhren (1, 2) abwechselnd aktiviert, um mit jeweils einer der beiden Röntgenröhren jeweils ein Bild zu erzeugen, sowie durch eine computerunterstützte Bildverarbeitungseinrichtung mit der
- eine Fläche bestimmt wird, die aufgespannt wird durch
   - die Sichtlinie von einer ersten Röntgenröhre zum Objekt in einem zu einem früheren Zeitpunkt aufgenommenen Bild und
   - die Sichtlinie von der ersten Röntgenröhre zu dem Objekt in einem zu einem späteren Zeitpunkt aufgenommenen Bild;
- ein Schnittpunkt bestimmt wird aus
   - einer Sichtlinie von der zweiten Röntgenröhre zu dem Objekt in einem Bild, das zu einem Zeitpunkt aufgenommen wird, der zwischen dem früheren und dem späteren Zeitpunkt liegt, und
   - der aufgespannten Fläche;
- eine weitere Fläche bestimmt wird, wobei der spätere Zeitpunkt zu einem neuen früheren Zeitpunkt wird, und wobei die weitere Fläche aufgespannt wird durch
   - die Sichtlinie von der ersten Röntgenröhre zum Objekt in einem zu dem neuen früheren Zeitpunkt aufgenommenen Bild und
   - die Sichtlinie von der ersten Röntgenröhre zu dem Objekt in einem zu einem neuen späteren Zeitpunkt aufgenommenen Bild;
- ein weiterer Schnittpunkt bestimmt wird aus
   - einer Sichtlinie von der zweiten Röntgenröhre zu dem Objekt in einem Bild, das zu einem Zeitpunkt aufgenommen wird, der zwischen dem neuen früheren und dem neuen späteren Zeitpunkt liegt, und
   - der weiteren aufgespannten Fläche;
- die räumliche Verbindungsgerade R12, welche die Schnittpunkte verbindet, berechnet wird; die Minimaltransversale zwischen der Verbindungsgeraden R12 und der Sichtlinie des Objektes zum neuen späteren Zeitpunkt errechnet wird; und
- aus dem Schnittpunkt der Minimaltransversalen und der Sichtlinie zum neuen späteren Zeitpunkt die dreidimensionale Position des verfolgten Objektes approximiert wird.

Natürlich können alle hierin beschriebenen Merkmale, auch wenn sie verfahrensmäßig formuliert sind, durch entsprechende Vorrichtungen und Einrichtungen umgesetzt werden.

Mit etwas anderen Worten kann die vorliegende Erfindung auch so beschrieben werden, dass sie einen Weg aufzeigt, um dreidimensionale Koordinaten eines beweglichen Objektes trotz der Abweichungen von Echtzeit-Projektionslinien zu ermitteln. Durch das Errechnen der Oberfläche, die durch eine Projektionslinie des Objekts in einem Bild und durch ihre direkte Vorgängerin bestimmt wird, und durch die Berechnung des Schnittpunktes dieser Oberfläche mit einer Sichtlinie bzw. Projektionslinie desselben Objektes, erfasst von dem anderen Sichtwinkel, also durch die andere Röntgenröhre, zu einem Zeitpunkt zwischen den beiden vorgenannten Bilderfassungen, kann ein dreidimensionaler Punkt bestimmt werden. Dieser rekonstruierte dreidimensionale Punkt ist eine Annäherung der realen Position des Objektes zu diesem vorgegebenen Zeitpunkt. Die Genauigkeit dieser Rekonstruktion hängt von der Linearität der 3D-Bewegung des Objektes ab. Dieser rekonstruierte Punkt entspricht aber einem Zeitpunkt in der Vergangenheit und wegen dieses Zeitunterschiedes ist ein Echtzeit-Tracking somit noch nicht möglich.

Wenn aber die oben beschriebene Rekonstruktion eines 3D-Punktes in der Folge nochmals durchgeführt wird, erhält man zwei 3D-Punkte, die jeweils auf einer Projektionslinie einer Röntgeneinheit liegen.

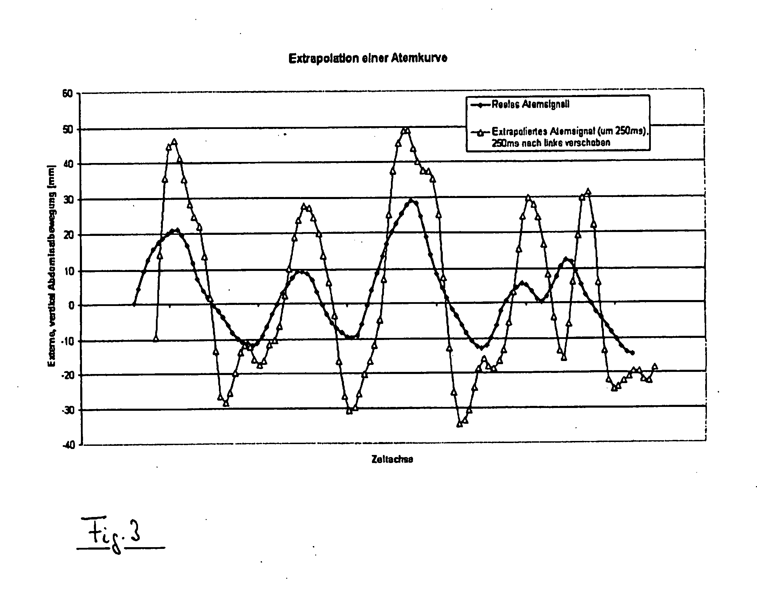

Eine Linie durch diese beiden rekonstruierten Punkte würde die Bewegungsbahn des Objektes durch den dreidimensionalen Raum annähern. Ein Problem hierbei liegt darin, dass eine solche Bewegungsbahn, insbesondere bei nicht linearen bzw. über die Zeit veränderlichen Bewegungen eine ausreichend genaue Echtzeit-Ortsbestimmung für den Punkt oft nicht gestattet. Die Figur 3 zeigt Kurven für ein reales Atemsignal (Punktkurve) sowie für ein extrapoliertes Atemsignal (Dreieckskurve). Aufgetragen ist die externe, vertikale Abdominalbewegung in mm über der Zeitachse, und aus diesen beiden Darstellungen wird deutlich, wie ungenau eine extrapolierte Atemkurve gegenüber der realen Atmungsverschiebung sein kann.

Kommt man nun zurück auf die durch zwei Punkte approximierte Bewegungsbahn des Objektes, so wird klar, dass eine solche approximierte Bahn alleine noch keine guten Ergebnisse bzw. Annäherung für ein Echtzeit-Tracking bieten kann.

Die vorliegende Erfindung hat aber erkannt, dass aus den angewendeten Schritten eine zusätzliche Information zur Verfügung steht, und zwar eine tatsächliche Echtzeit-Information über die Lage des Objektes. Das Objekt liegt nämlich direkt auf der Sichtlinie der letzten Röntgenprojektion, die für die Flächenaufspannung verwendet wird, und zwar genau zum Zeitpunkt des dabei erstellten Röntgenbildes. Man kann diese Information verwerten, um die exakte Position des Objektes für den letzten Zeitpunkt, an dem eine Projektions-Sichtlinie zur Verfügung steht, sehr genau anzunähern, und hierfür wird der kürzeste Abstand zwischen der Bewegungsbahn und der letzten Sichtlinie mit einem Lot auf die Sichtlinie bestimmt. Dieses Lot ist die so genannte Minimaltransversale. An der Stelle, wo die Minimaltransversale, d.h. die kürzeste (senkrechte) Verbindung zwischen den beiden genannten windschief zueinander liegenden Geraden, auf die letzte Projektions-Sichtlinie trifft, liegt wiederum ein Punkt, und gerade dieser Punkt liegt in sehr guter Annäherung an der realen Echtzeit-Position des Objektes, d.h. zu der Zeit, zu der die letzte Röntgensichtlinie zur Flächenaufspannung erstellt wurde.

Die Präzision des Verfahrens wird abhängig von der Linearität der Objektbewegung etwas variieren. Speziell für bewegliche Objekte wie implantierte röntgensichtbare Marker, die herkömmlicherweise für ein Strahlungstherapie-Tracking verwendet werden, lässt sich eine Hauptbewegungsachse bestimmen, die typischerweise eine Bewegung entlang der Körperlängsachse für Implantate in der Lunge, in der Leber oder in den Nieren ist. In diesen Fällen ist es speziell von Wichtigkeit, eine gute räumliche/zeitliche Auflösung für das Objekt entlang dieser Richtung zu haben. Deshalb sollten bevorzugt die Sichtlinien beider Röntgeneinheiten (Röntgendetektoren) diese Hauptbewegungsachse unter einem ausreichend großen Winkel schneiden, so dass die Hauptbewegungskomponente gleich gut in Bildern beider Röntgenquellen/Detektoren-Paare erfasst werden kann.

Die Erfindung wird im Weiteren anhand eines Ausführungsbeispiels und mit Hilfe der beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: eine schematische Darstellung der Punkt- und Flächenakquirierung für die erfindungsgemäße Röntgenverfolgung;
- Figur 2: eine schematische Darstellung der Echtzeit-Objektortbestimmung mit Hilfe einer Minimaltransversalen; und
- Figur 3: eine graphische Darstellung, die die Abweichung zwischen extrapolierter und realer Atemkurve zeigt.

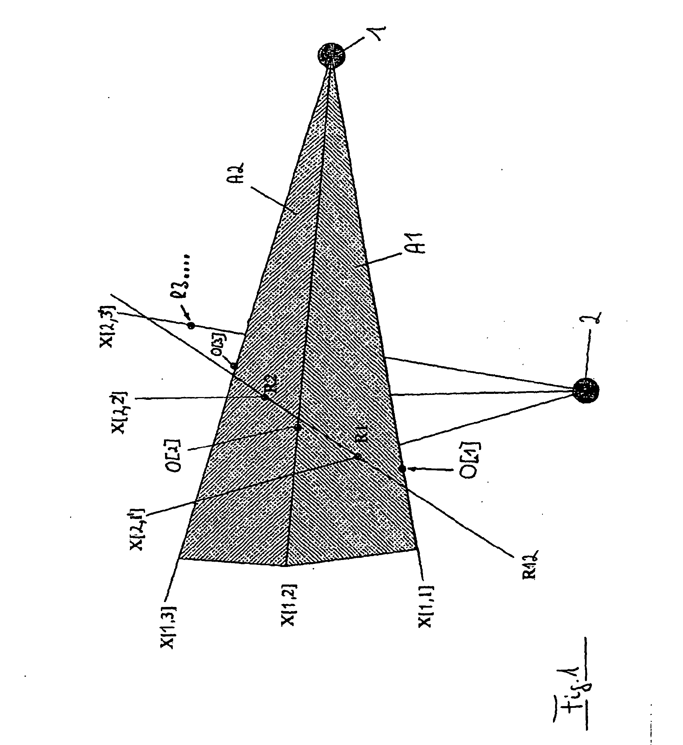

In der Figur 1 sind zwei Röntgenquellen dargestellt, die mit den Bezugszeichen 1 und 2 versehen sind. Ferner zeigt die Figur ein Objekt O, das sich bewegt, und zwar zu mehreren hintereinander liegenden Zeitpunkten, also das Objekt O[1] zum Zeitpunkt 1, das Objekt O[2] zum Zeitpunkt 2 und das Objekt O[3] zum Zeitpunkt 3. Die erfindungsgemäße Positionsverfolgung wird wie folgt durchgeführt:

Zum Zeitpunkt 1 wird mit der Röntgenquelle 1 ein erstes Röntgenbild erstellt, und eine Sichtlinie X[1, 1] in diesem Röntgenbild geht durch das Objekt O[1]. Das Objekt bewegt sich danach weiter und kommt zum Zeitpunkt 2 an die Stelle, an der es mit O[2] dargestellt ist. Auch zu diesem Zeitpunkt wird wiederum ein Röntgenbild durch die Quelle 1 erstellt, und man erhält die Sichtlinie X[1, 2].

Zwischen den Zeitpunkten 1 und 2 (bei dieser Ausführungsform nach der Hälfte der vergangenen Zeit) wird eine Röntgenaufnahme mit der Röntgenquelle 2 und der Sichtlinie X[2, 1'] erstellt, also zu dem Zwischenzeitpunkt 1'. Wenn man nun zwischen den Sichtlinien X[1, 1] und X[1, 2] die Fläche A1 berechnet bzw. aufspannt, kann man auch den Punkt ausrechnen, an dem die Objekt-Sichtlinie X[2, 1'] von der Röntgenquelle 2 zum Zwischenzeitpunkt 1' die Fläche A1 durchstößt und man erhält den Punkt R1. Dieser Schnittpunkt R1 kann als ein erster angenähert rekonstruierter Punkt auf der Bahn des Objektes angesehen werden, aber diese Information ist nur für einen Zeitpunkt in der Vergangenheit gültig, da sich das Objekt sich inzwischen schon bei O[2] befindet.

Die Information über R1 kann aber trotzdem verwendet werden, wenn der vorliegende Ablauf nochmals durchgeführt wird, d.h. zu einem Zeitpunkt 3 wird wiederum mit der Röntgenröhre 1 die Sichtlinie X[1, 3] aufgenommen, auf der der Punkt O[3] zum Zeitpunkt 3 liegt. Wenn nun wiederum ein Bild mit der Röntgenröhre 2 und der Sichtlinie X[2, 2'] für den Zwischenzeitpunkt 2' aufgenommen wird und wiederum der Punkt R2 bestimmt wird, an dem die Sichtlinie X[2, 2'] die Fläche A2 durchstößt (Fläche zwischen X[1, 2] und X[1, 3]), hat man schon zwei extrapolierte Punkte R1 und R2 und kann somit eine angenäherte Bewegungsbahn für das Objekt bestimmen. Diese angenäherte Bewegungsbahn ist in Figur 1 mit R12 gezeigt; sie ist eine Gerade durch R1 und R2. Um die Bewegungsbahn weiter angenähert fortsetzen zu können, können diese Vorgänge wiederholt werden, und deshalb ist in der Figur 1 auch noch die Sichtlinie X[2, 3'] angedeutet, auf der dann der Punkt R3 liegen würde. Der Vorgang wird in dieser Folge fortgesetzt.

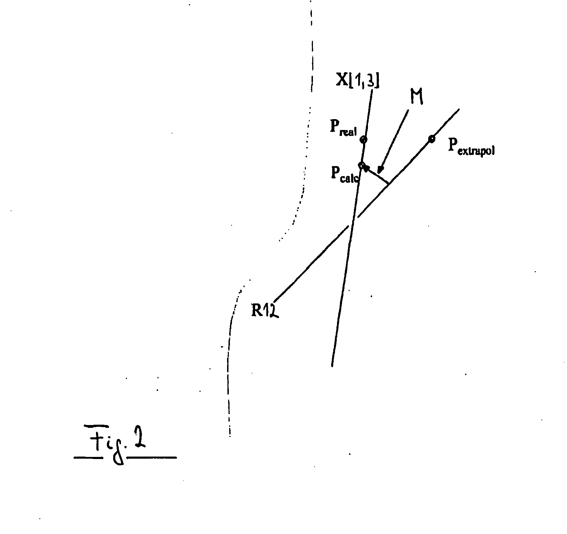

Weil die lineare Annäherung der Bewegungsbahn R12 relativ mangelhaft sein kann, wie aus den Abweichungen in der realen und extrapolierten Atemkurve in Figur 3 hervorgeht und wie oben schon beschrieben wurde, wäre die Echtzeit-Objektpunktberechnung anhand der Bewegungsbahn R12 zwar möglich, jedoch noch ungenau. Die Erfindung hat aber erkannt, dass bei dem aufgezeigten Vorgehen schon eine Information vorliegt, die eine Verbesserung der Genauigkeit gestattet, und zwar die Information, dass das Objekt O zum Zeitpunkt 3 gerade auf der Sichtlinie X[1, 3] liegt. Diese Information lässt sich nun intelligent auswerten, und zur Erläuterung wird auf die Figur 2 Bezug genommen.

Die Figur 2 zeigt wiederum die Bewegungsbahn R12. Zum Vergleich ist auf der Bewegungsbahn R12 einmal der Punkt Pₑₓₜᵣₐₚₒₗ aufgezeigt, der sich ergeben würde, wenn man auf dieser Bewegungsbahn lediglich fortgesetzt die Position des Objektes zum Zeitpunkt 3 berechnen würde. Die tatsächliche, erfindungsgemäße Berechnung geht aber einen anderen Weg, indem sie die Information verarbeitet, dass der tatsächliche Objektort Pᵣₑₐₗ auf der Sichtlinie X[1, 3] liegen muss. Obwohl durch diese eine Projektion nicht exakt feststellbar ist, an welchem Längenort der Punkt Pᵣₑₐₗ auf X[1, 3] liegt, lässt sich doch die Information über die Lage dieser Sichtlinie auswerten, und zwar indem eine Minimaltransversale M zwischen den beiden windschiefen Geraden R12 und X[1, 3] berechnet wird. Die Minimaltransversale ist der kürzeste Abstand zwischen den beiden Geraden im Raum, und sie schneidet die beiden Geraden senkrecht. Dort wo die Minimaltransversale M die Sichtlinie X[1, 3] schneidet, ergibt sich der Punkt P_{calc}, und dieser Punkt P_{calc} ist sehr gut an die reale Position des Punktes Pᵣₑₐₗ angenähert. Der Punkt P_{calc} ist also im Ergebnis die sehr gut approximierte Echtzeitposition des Objektes O zum Zeitpunkt 3.

Es lässt sich somit durch die erfindungsgemäße Objektverfolgung eine sehr gute Positionsannäherung bzw. Bestimmung für ein Objekt bei wesentlich reduzierter Strahlenbelastung und mit den anderen oben genannten Vorteilen realisieren.

## Patentansprüche

1. Nicht diagnostisches, stereoskopisches Röntgenverfolgungsverfahren zur Verfolgung bewegter Objekte im Rahmen der Radiotherapie und Radiochirurgie, bei dem mit zwei Röntgenröhren (1, 2) wiederholt Röntgenbilder eines Objekts (O) entlang zweier verschiedener, sich unter einem bekannten Winkel schneidender Sichtlinien durch das Zielgebiet eines Bestrahlungsgerätes aufgenommen werden, **dadurch gekennzeichnet, dass**
a) die Ansteuerung der Röntgenröhren (1, 2) so erfolgt, dass abwechselnd mit einer der beiden Röntgenröhren jeweils ein Bild erzeugt wird; und computerunterstützt
b) eine Fläche (A1) bestimmt wird, die aufgespannt wird durch
- die Sichtlinie (X[1, 1]) von einer ersten Röntgenröhre (1) zum Objekt (O[1]) in einem zu einem früheren Zeitpunkt (t1) aufgenommenen Bild und
- die Sichtlinie (X[1, 2]) von der ersten Röntgenröhre (1) zu dem Objekt (O[2]) in einem zu einem späteren Zeitpunkt (t2) aufgenommenen Bild;
c) ein Schnittpunkt (R1) bestimmt wird aus
- einer Sichtlinie von der zweiten Röntgenröhre (2) zu dem Objekt (O) in einem Bild, das zu einem Zeitpunkt (t1') aufgenommen wird, der zwischen dem früheren und dem späteren Zeitpunkt liegt, und
- der aufgespannten Fläche (A1);
d) eine weitere Fläche (A2) bestimmt wird, wobei der spätere Zeitpunkt (t2) zu einem neuen früheren Zeitpunkt (t2) wird, und wobei die weitere Fläche aufgespannt wird durch
- die Sichtlinie (X[1, 2]) von der ersten Röntgenröhre (1) zum Objekt (O[2]) in einem zu dem neuen früheren Zeitpunkt (t2) aufgenommenen Bild und
- die Sichtlinie (X[1, 3]) von der ersten Röntgenröhre (1) zu dem Objekt (O[3]) in einem zu einem neuen späteren Zeitpunkt (t3) aufgenommenen Bild;
e) ein weiterer Schnittpunkt (R2) bestimmt wird aus
- einer Sichtlinie von der zweiten Röntgenröhre (2) zu dem Objekt (O) in einem Bild, das zu einem Zeitpunkt (t2') aufgenommen wird, der zwischen dem neuen früheren und dem neuen späteren Zeitpunkt (t3) liegt, und
- der weiteren aufgespannten Fläche (A2);
f) die räumliche Verbindungsgerade (R12), welche die Schnittpunkte (R1 und R2) verbindet, berechnet wird;
g) die Minimaltransversale zwischen der Verbindungsgeraden (R12) und der Sichtlinie (X[1, 3]) des Objektes (O[3]) zum neuen späteren Zeitpunkt (t3) errechnet wird; und
h) aus dem Schnittpunkt der Minimaltransversalen (M) und der Sichtlinie (X(1, 3]) zum neuen späteren Zeitpunkt (t3) die dreidimensionale Position (P_{calc}) des verfolgten Objektes (O) approximiert wird.

2. Verfahren nach Anspruch 1, bei dem der zeitliche Abstand zwischen dem Erzeugen eines Bildes mit der ersten und zweiten Röntgenröhre (1, 2) so eingestellt wird, dass in einem regelmäßigen Zeitabstand (T) jeweils abwechselnd ein Bild mit der ersten und zweiten Röhre (1, 2) erzeugt wird.

3. Verfahren nach Anspruch 1 oder 2, bei dem das verfolgte Objekt ein Markierungsimplantat ist, insbesondere ein Implantat, das sich korreliert zur Atembewegung bewegt.

4. Verfahren nach Anspruch 1 oder 2, bei dem das verfolgte Objekt eine röntgenabbildbare Körperstruktur oder Körperlandmarke ist, insbesondere eine Struktur oder Landmarke, die sich korreliert zur Atembewegung bewegt.

5. Verfahren nach einem der Ansprüche 1 bis 4, bei dem die Länge der Minimaltransversalen mit einem vorgegebenen Schwellwert verglichen wird, wobei beim Überschreiten des Schwellwertes ein Zustand der Ungenauigkeit angenommen und eine Verfahrensanpassung vorgenommen wird, insbesondere eine Erhöhung der Abtastrate, die durch eine Verkleinerung der Zeitabstände zwischen den Bildern realisiert wird.

6. Verfahren nach einem der Ansprüche 1 bis 4, bei dem die Länge der Minimaltransversalen mit einem vorgegebenen Schwellwert verglichen wird, wobei beim Überschreiten des Schwellwertes ein Zustand der Ungenauigkeit angenommen und eine Fehlermeldung ausgegeben wird.

7. Programm, das, wenn es auf einem Computer läuft oder in einem Computer geladen ist, den Computer veranlasst, ein Verfahren gemäß einem der Ansprüche 1 bis 6 durchzuführen.

8. Computerprogramm-Speichermedium, das ein Programm nach Anspruch 7 aufweist.

9. Stereoskopische Röntgenverfolgungsvorrichtung zur Verfolgung bewegter Objekte im Rahmen der Radiotherapie und Radiochirurgie, mit zwei Röntgenröhren (1, 2), die wiederholt Röntgenbilder eines Objekts (O) entlang zweier verschiedener, sich unter einem bekannten Winkel schneidender Sichtlinien durch das Zielgebiet eines Bestrahlungsgerätes aufnehmen, **gekennzeichnet durch** eine Steuerungseinrichtung, welche die Röntgenröhren (1, 2) abwechselnd aktiviert, um mit jeweils einer der beiden Röntgenröhren jeweils ein Bild zu erzeugen, sowie **durch** eine computerunterstützte Bildverarbeitungseinrichtung mit der
- eine Fläche (A1) bestimmt wird, die aufgespannt wird **durch**
- die Sichtlinie (X[1, 1]) von einer ersten Röntgenröhre (1) zum Objekt (O[1]) in einem zu einem früheren Zeitpunkt (t1) aufgenommenen Bild und
- die Sichtlinie (X[1, 2]) von der ersten Röntgenröhre (1) zu dem Objekt (O[2]) in einem zu einem späteren Zeitpunkt (t2) aufgenommenen Bild;
- ein Schnittpunkt (R1) bestimmt wird aus
- einer Sichtlinie von der zweiten Röntgenröhre (2) zu dem Objekt (O) in einem Bild, das zu einem Zeitpunkt (t1') aufgenommen wird, der zwischen dem früheren und dem späteren Zeitpunkt liegt, und
- der aufgespannten Fläche (A1);
- eine weitere Fläche (A2) bestimmt wird, wobei der spätere Zeitpunkt (t2) zu einem neuen früheren Zeitpunkt (t2) wird, und wobei die weitere Fläche aufgespannt wird durch
- die Sichtlinie (X[1, 2]) von der ersten Röntgenröhre (1) zum Objekt (O[2]) in einem zu dem neuen früheren Zeitpunkt (t2) aufgenommenen Bild und
- die Sichtlinie (X[1, 3]) von der ersten Röntgenröhre (1) zu dem Objekt (O[3]) in einem zu einem neuen späteren Zeitpunkt (t3) aufgenommenen Bild;
- ein weiterer Schnittpunkt (R2) bestimmt wird aus
- einer Sichtlinie von der zweiten Röntgenröhre (2) zu dem Objekt (O) in einem Bild, das zu einem Zeitpunkt (t2') aufgenommen wird, der zwischen dem neuen früheren und dem neuen späteren Zeitpunkt (t3) liegt, und
- der weiteren aufgespannten Fläche (A2);
- die räumliche Verbindungsgerade (R12), welche die Schnittpunkte (R1 und R2) verbindet, berechnet wird;
- die Minimaltransversale zwischen der Verbindungsgeraden (R12) und der Sichtlinie (X[1, 3]) des Objektes (O[3]) zum neuen späteren Zeitpunkt (t3) errechnet wird; und
- aus dem Schnittpunkt der Minimaltransversalen (M) und der Sichtlinie (X[1, 3]) zum neuen späteren Zeitpunkt (t3) die dreidimensionale Position (R_{calc}) des verfolgten Objektes (O) approximiert wird.

## Claims

1. A non-diagnostic, stereoscopic x-ray tracking method for tracking moving objects in the context of radiotherapy and radiosurgery, in which two x-ray tubes (1, 2) are used to repeatedly record x-ray images of an object (O) along two different viewing lines through the target area of an irradiating apparatus, said viewing lines intersecting at a known angle, **characterised in that:**
a) the x-ray tubes (1, 2) are controlled such that one image is generated in each case, alternately using one of the two x-ray tubes; and with the assistance of a computer:
b) a surface (A1) is determined which is spanned by
- the viewing line (X[1, 1]) from a first x-ray tube (1) to the object (O[1]) in an image recorded at an earlier point in time (t1) and
- the viewing line (X[1, 2]) from the first x-ray tube (1) to the object (O[2]) in an image recorded at a later point in time (t2);
c) an intersecting point (R1) is determined from
- a viewing line from the second x-ray tube (2) to the object (O) in an image recorded at a point in time (t1') between the earlier point in time and the later point in time, and
- the spanned surface (A1);
d) another surface (A2) is determined, wherein the later point in time (t2) becomes a new earlier point in time (t2), and wherein said other surface is spanned by
- the viewing line (X[1, 2]) from the first x-ray tube (1) to the object (O[2]) in an image recorded at the new earlier point in time (t2) and
- the viewing line (X[1, 3]) from the first x-ray tube (1) to the object (O[3]) in an image recorded at a new later point in time (t3);
e) another intersecting point (R2) is determined from
- a viewing line from the second x-ray tube (2) to the object (O) in an image recorded at a point in time (t2') between the new earlier point in time and the new later point in time (t3), and
- the other spanned surface (A2);
f) the spatial straight connecting line (R12), which connects the intersecting points (R1 and R2), is calculated;
g) the minimum transversal between the straight connecting line (R12) and the viewing line (X[1, 3]) of the object (O[3]) at the new later point in time (t3) is calculated; and
h) the three-dimensional position (P_{calc}) of the tracked object (O) is approximated from the intersecting point of the minimum transversal (M) and the viewing line (X[1, 3]) at the new later point in time (t3).

2. The method according to claim 1, wherein the time interval between generating an image using the first and second x-ray tube (1, 2) is set such that one image is alternately generated using the first and second tube (1, 2), at regular time intervals (T) in each case.

3. The method according to claim 1 or 2, wherein the tracked object is a marking implant, in particular an implant which moves in correlation with the movement of breathing.

4. The method according to claim 1 or 2, wherein the tracked object is a body structure or body landmark which can be mapped using x-rays, in particular a structure or landmark which moves in correlation with the movement of breathing.

5. The method according to any one of claims 1 to 4, wherein the length of the minimum transversal is compared with a predetermined threshold value, wherein if the threshold value is exceeded, a state of imprecision is assumed and the method is adapted, in particular by increasing the scanning rate, which is realised by reducing the time intervals between the images.

6. The method according to any one of claims 1 to 4, wherein the length of the minimum transversal is compared with a predetermined threshold value, wherein if the threshold value is exceeded, a state of imprecision is assumed and an error message is outputted.

7. A program which, when it is running on a computer, causes the computer to perform a method in accordance with any one of claims 1 to 6.

8. A computer program storage medium comprising a program according to claim 7.

9. A stereoscopic x-ray tracking device for tracking moving objects in the context of radiotherapy and radiosurgery, comprising two x-ray tubes (1, 2) which repeatedly record x-ray images of an object (O) along two different viewing lines through the target area of an irradiating apparatus, said viewing lines intersecting at a known angle, **characterised by** a control means which alternately activates the x-ray tubes (1, 2) in order to generate one image in each case, respectively using one of the two x-ray tubes, and **characterised by** a computer-assisted image processing means, using which:
- a surface (A1) is determined which is spanned by
- the viewing line (X[1, 1]) from a first x-ray tube (1) to the object (O[1]) in an image recorded at an earlier point in time (t1) and
- the viewing line (X[1, 2]) from the first x-ray tube (1) to the object (O[2]) in an image recorded at a later point in time (t2);
- an intersecting point (R1) is determined from
- a viewing line from the second x-ray tube (2) to the object (O) in an image recorded at a point in time (t1') between the earlier point in time and the later point in time, and
- the spanned surface (A1);
- another surface (A2) is determined, wherein the later point in time (t2) becomes a new earlier point in time (t2), and wherein said other surface is spanned by
- the viewing line (X[1, 2]) from the first x-ray tube (1) to the object (O[2]) in an image recorded at the new earlier point in time (t2) and
- the viewing line (X[1, 3]) from the first x-ray tube (1) to the object (O[3]) in an image recorded at a new later point in time (t3);
- another intersecting point (R2) is determined from
- a viewing line from the second x-ray tube (2) to the object (O) in an image recorded at a point in time (t2') between the new earlier point in time and the new later point in time (t3), and
- the other spanned surface (A2);
- the spatial straight connecting line (R12), which connects the intersecting points (R1 and R2), is calculated;
- the minimum transversal between the straight connecting line (R12) and the viewing line (X[1, 3]) of the object (O[1]) at the new later point in time (t3) is calculated; and
- the three-dimensional position (P_{calc}) of the tracked object (O) is approximated from the intersecting point of the minimum transversal (M) and the viewing line (X[1, 3]) at the new later point in time (t3).

## Revendications

1. Procédé de radioscopie stéréoscopique non diagnostique pour suivre des objets en mouvement, dans le cadre de la radiothérapie et de la radiochirurgie, dans lequel, à l'aide de deux tubes à rayons X (1, 2), on enregistre, de manière renouvelée, des images radio d'un objet (O) le long de deux lignes de visée se coupant suivant un angle connu, à travers la région cible d'un appareil d'irradiation,
**caractérisé en ce que**
a) la commande des tubes à rayons X (1, 2) s'effectue **en ce qu'**il est produit alternativement chaque fois une image avec l'un des deux tubes à rayons X ; et de manière assistée par ordinateur
b) on détermine une surface (A1) qui est définie par
- la ligne de visée (X [1,1]) d'un premier tube à rayons X (1) vers l'objet (O [1]) dans une image prise à un instant antérieur (t1) et
- la ligne de visée (X [1,2]) du premier tube à rayons X (1) vers l'objet (O [2]) dans une image prise à un instant ultérieur (t2) ;
c) on détermine un point d'intersection (R1) à partir
- d'une ligne de visée du second tube à rayons X (2) vers l'objet (O) dans une image qui est prise à un instant (t1') qui se situe entre l'instant antérieur et l'instant ultérieur, et
- la surface (A1) définie.
d) on détermine une autre surface (A2), l'instant ultérieur (t2) devenant un nouvel instant antérieur (t2), et l'autre surface étant définie par
- la ligne de visée (X [1,2]) du premier tube à rayons X (1) vers l'objet (O [2]) dans une image prise au nouvel instant antérieur (t2), et
- la ligne de visée (X [1,3]) du premier tube à rayons X (1) vers l'objet (O [3]) dans une image prise à un nouvel instant ultérieur (t3) ;
e) on détermine un autre point d'intersection (R2) à partir
- d'une ligne de visée du second tube à rayons X (2) vers l'objet (O) dans une image qui est prise à un instant (t2') qui se situe entre le nouvel instant antérieur et le nouvel instant ultérieur (t3), et
- de l'autre surface (A2) définie ;
f) on calcule la droite de liaison spatiale (R12), qui relie les points d'intersection (R1 et R2) ;
g) on calcule la transversale minimale entre les droites de liaison (R12) et la ligne de visée (X [1,3]) de l'objet (O [3]) au nouvel instant ultérieur (t3) ; et
h) à partir du point d'intersection des transversales minimales (M) et de la ligne de visée (X [1,3]) on approche au nouvel instant ultérieur (t3) la position tridimensionnelle (Pcalc) de l'objet (O) poursuivi.

2. Procédé selon la revendication 1, dans lequel l'intervalle temporel entre la production d'une image avec le premier et le second tubes à rayons X (1, 2) est réglé de manière que dans un intervalle de temps (T) régulier il soit produit alternativement une image avec le premier et le second tube (1, 2).

3. Procédé selon l'une quelconque des revendications 1 ou 2, dans lequel l'objet suivi est un implant de repérage, en particulier un implant qui se déplace de manière corrélée au mouvement respiratoire.

4. Procédé selon l'une quelconque des revendications 1 ou 2, dans lequel l'objet suivi est une structure corporelle ou repère corporel pouvant être radiographié, en particulier une structure ou un repère qui se déplace de manière corrélée au mouvement respiratoire.

5. Procédé selon l'une quelconque des revendications 1 à 4, dans lequel la longueur des transversales minimales est comparée à une valeur de seuil prédéfinie, lors du dépassement de la valeur de seuil un état d'imprécision étant admis et une adaptation du procédé étant effectuée, en particulier une augmentation de la fréquence de balayage qui est obtenue par une réduction des intervalles de temps entre les images.

6. Procédé selon l'une quelconque des revendications 1 à 4, dans lequel on compare la longueur des transversales minimales à une valeur de seuil prédéfinie et lors du dépassement de la valeur de seuil on admet un état d'imprécision, et un signal de défaut est délivré.

7. Programme qui lorsqu'il tourne sur un ordinateur provoque la mise en oeuvre par l'ordinateur d'un procédé selon l'une quelconque des revendications 1 à 6.

8. Support de mémoire de programme d'ordinateur qui comporte un programme selon la revendication 7.

9. Dispositif de radioscopie stéréoscopique pour la poursuite d'objets en mouvement dans le cadre de la radiothérapie et de la radiochirurgie, avec deux tubes à rayons X (1, 2) qui enregistre de manière renouvelée des images radio d'un objet (O) le long de deux lignes de visée différentes, se coupant suivant un angle connu, à travers la région cible d'un appareil d'irradiation, **caractérisé par** un dispositif de commande qui active alternativement les tubes à rayons X (1, 2), afin de produire chaque fois une image avec l'un des deux tubes à rayons X, ainsi que par un dispositif de traitement d'image assisté par ordinateur avec lequel
- on détermine une surface (A1) qui est définie par
- la ligne de visée (X [1,1]) d'un premier tube à rayons X (1) vers l'objet (O [1]) dans une image prise à un instant antérieur (t1) et
- la ligne de visée (X [1,2]) du premier tube à rayons X (1) vers l'objet (O [2]) dans une image prise à un instant ultérieur (t2) ;
- on détermine un point d'intersection (R1) à partir
- d'une ligne de visée du second tube à rayons X (2) vers l'objet (O) dans une image qui est prise à un instant (t1') qui se situe entre l'instant antérieur et l'instant ultérieur, et
- de la surface (A1) définie ;
- on détermine une autre surface (A2) l'instant ultérieur (t2) devenant un nouvel instant antérieur (t2)et l'autre surface étant définie par
- la ligne de visée (X [1,2]) du premier tube à rayons X (1) vers l'objet (O [2]) dans une image prise au nouvel instant antérieur (t2), et
- la ligne de visée (X [1,3]) du premier tube à rayons X (1) vers l'objet (O [3]) dans une image prise à un nouvel instant ultérieur (t3) ;
- on détermine un autre point d'intersection (R2) à partir
- d'une ligne de visée du second tube à rayons X (2) vers l'objet (O) dans une image qui est prise à un instant (t2') qui se situe entre le nouvel instant antérieur et le nouvel instant ultérieur (t3), et
- de l'autre surface (A2) définie ;
- on calcule la droite de liaison spatiale (R12) qui relie les points d'intersection (R1 et R2) ;
- on calcule la transversale minimale entre les droites de liaison (R12) et la ligne de visée (X [1,3]) de l'objet (O [3]) au nouvel instant ultérieur (t3) ; et
- à partir du point d'intersection des transversales minimales (M) et de la ligne de visée (X [1,3]) on approche au nouvel instant ultérieur (t3) la position tridimensionnelle (Pcalc) de l'objet (O) poursuivi.
